# EUROPEAN PATENT SPECIFICATION

(11) **EP 3 634 513 B1**
(45) Date of publication and mention of the grant of the patent: **30.06.2021**
(21) Application number: 18732991.7
(22) Date of filing: 11.05.2018
(51) Int. Cl.: A61L 27/26, A61L 27/20, A61L 27/24

(54) **ADHERENT, BIORESORBABLE TRANSPLANT SUBSTRATE**
ADHÄSIVES, BIORESORBIERBARES TRANSPLANTATSUBSTRAT
SUBSTRAT DE GREFFE ADHÉSIF, BIORÉSORBABLE

(30) Priority: 12.05.2017 US 201762505696 P
(43) Date of publication of application: 15.04.2020
(73) Proprietor: KCI USA, Inc., San Antonio, TX 78265 (US); Systagenix Wound Management, Limited, Beehive Ring Road, Gatwick Airport, West Sussex RH6 0PA (GB)
(72) Inventor: WAITE, Alexander, Keighley North Yorkshire BD22 0FN (GB); DELURY, Craig, San Antonio, TX 78265 (US); THISTLETHWAITE, Joy, Kelbrook Barnoldswick BB18 6LW (GB)
(74) Representative: Simmons & Simmons
(86) International application number: PCT/US2018/032312
(87) International publication number: WO 2018/209230

(56) References cited:
- WO-A1-2004/112850
- US-A1- 2013 204 273
- US-A1- 2014 277 454
- US-A1- 2016 374 704

## Description

### TECHNICAL FIELD

The presently-disclosed subject matter generally relates to tissue treatment systems and more particularly, to devices for cultivating and transplanting a tissue graft, for example, a skin tissue graft.

### BACKGROUND

Skin is the largest organ of the human body, representing approximately 16% of a person's total body weight. Because it interfaces with the environment, skin has an important function in body defense, acting as an anatomical barrier from pathogens and other environmental substances. Skin also provides a semi-permeable barrier that prevents excessive fluid loss while ensuring that essential nutrients are not washed out of the body. Other functions of skin include insulation, temperature regulation, and sensation. Skin may be subject to many forms of damage, including burns, trauma, disease, and depigmentation (e.g., vitiligo).

Skin grafts are often used to repair such skin damage. Skin grafting is a surgical procedure in which a section of skin is removed from one area of a person's body (*e.g.,* an autograft), removed from another human source (*e.g.,* an allograft), or removed from another animal (*e.g.,* a xenograft), and transplanted to a recipient site of a patient, such as a wound site or abnormality site. As with any surgical procedure, skin grafting involves certain risks. Complications associated with skin grafting may include graft failure, rejection of the skin graft, bleeding, and fluid accumulation or infection at either the donor or recipient site. In some scenarios, it may be preferable to use an autograft instead of an allograft or a xenograft, for example, to reduce complications, such as graft failure or rejection.

A problem encountered when using an autograft is that skin is taken from another area of a person's body to produce the graft, resulting in trauma and wound generation at the donor site. Generally, the size of the graft matches the size of the recipient site, and thus a large recipient site requires removal of a large section of skin from a donor site, leading to increased pain and discomfort and longer healing time. Additionally, as the size of the section of skin removed from the donor site increases, so does the possibility of infection.

US 2014/0277454 A1 concerns methods and devices for management of fluids during skin graft transplantation.

Due to risks associated with skin grafting for larger tissue sites, techniques have been developed for harvesting a large number of smaller grafts, sometimes called micrografts, to reduce the trauma at the donor site. Nevertheless, there remains a need for improvements to these techniques and the devices involved when harvesting micrografts from a donor site and/or transferring the micrografts to the recipient site, for example, substrates which may be employed in transplanting micrografts and that make possible improved healing at that recipient site.

### SUMMARY

The scope of protection is defined by the claims. Any references in the description to methods of treatments refer to the products of the present invention (substrates and systems) for use in a method for treatment of the human or animal body by therapy. Any "embodiment" or "example" which is disclosed in the description but is not covered by the claims should be considered as presented for illustrative purpose only.

Systems and compositions in the form of a substrate for transplantation of a skin micrograft are set forth in the appended claims. Illustrative embodiments are also provided to enable a person skilled in the art to make and use the claimed subject matter.

Disclosed herein are one or more embodiments of a substrate for transplanting a skin micrograft. The substrate may be may be configured to receive the tissue micrograft and be positioned at a recipient site. The substrate may have a first surface and a second surface, and comprises a bioresorbable material and an adhesive. The bioresorbable material may comprise oxidized regenerated cellulose (ORC), for example, from about 40% to about 50% by weight of the substrate. The bioresorbable material may comprise collagen, for example, from about 50% to about 60% by weight of the substrate. The adhesive comprises a sugar, for example, glucose from about 8% to about 16% by weight of the substrate.

Also disclosed herein are systems for transplanting a tissue micrograft. The system comprises a tissue micrograft-harvesting device. The tissue micrograft-harvesting device may comprise a base-plate having a plurality of apertures. The system further comprises a substrate which is configured to receive the tissue micrograft and be positioned at a recipient site. The substrate may have a first surface and a second surface, and comprises a bioresorbable material and an adhesive. The bioresorbable material may comprise oxidized regenerated cellulose (ORC), for example, from about 40% to about 50% by weight of the substrate. The bioresorbable material may comprise collagen, for example, from about 50% to about 60% by weight of the substrate. The adhesive comprises a sugar, for example, glucose from about 8% to about 16% by weight of the substrate.

Also disclosed herein are methods for transplanting a tissue micrograft. The method may comprise forming a tissue microdome. The method may further comprise excising the tissue microdome from the donor site to form the tissue micrograft. The method may still further comprise transplanting the tissue micrograft from the donor site to a recipient site with a substrate to which the tissue micrograft is adhered. In some embodiments, the substrate may have a first surface and a second surface, and comprises a bioresorbable material and an adhesive. The bioresorbable material may comprise oxidized regenerated cellulose (ORC), for example, from about 40% to about 50% by weight of the substrate. The bioresorbable material may comprise collagen, for example, from about 50% to about 60% by weight of the substrate. The adhesive comprises a sugar, for example, glucose from about 8% to about 16% by weight of the substrate.

Objectives, advantages, and compositions, materials, systems, methods and a preferred mode of making and using the claimed subject matter may be understood best by reference to the accompanying drawings in conjunction with the following non-limiting detailed description of illustrative embodiments.

### BRIEF DESCRIPTION OF THE DRAWINGS

A more complete understanding of this specification may be obtained by reference to the following detailed description when taken in conjunction with the accompanying drawings.
Figure 1a is a schematic, perspective view of an example embodiment of a skin micrograft-harvesting device, according to an exemplary embodiment
Figure 1b is a schematic, perspective top view of the skin micrograft-harvesting device of Figure 1a with a head component removed and a cutter mechanism exposed, according to an exemplary embodiment.
Figure 2a is a schematic, perspective top view of the skin micrograft-harvesting device of Figure 1a with a transfer substrate deployed in the harvesting device to capture skin micrografts, according to an exemplary embodiment.
Figure 2b is a schematic, side view of the transfer substrate of Figure 2a, according to an exemplary embodiment.
Figure 2c is a schematic, perspective bottom view of the transfer substrate of Figure 2a, according to an exemplary embodiment.

### DETAILED DESCRIPTION

The example embodiments may also be described herein with reference to spatial relationships between various elements or to the spatial orientation of various elements depicted in the attached drawings. In general, such relationships or orientation assume a frame of reference consistent with or relative to a patient in a position to receive treatment. However, as should be recognized by those skilled in the art, this frame of reference is merely a descriptive expedient rather than a strict prescription.

### Skin Micrograft-Harvesting System

Disclosed herein are one or more embodiments of systems useful in a procedure to transplant a tissue micrograft, more particularly, a skin micrograft, for example, from a first tissue site, for example, a "donor site," to a second tissue site, for example, a "recipient site". In some embodiments, such a system generally comprises a skin micrograft-harvesting device and a substrate, which may also be referred to as a "support substrate" or "transfer substrate," as will likewise be disclosed herein, as well as one or more embodiments of compositions, suitable to form such a substrate.

The term "tissue site" as used herein may refer to a wound, defect, or other site located on or within tissue, including but not limited to, bone tissue, adipose tissue, muscle tissue, neural tissue, dermal tissue, vascular tissue, connective tissue, cartilage, tendons, or ligaments. A wound may include chronic, acute, traumatic, subacute, and dehisced wounds, partial-thickness burns, ulcers, such as diabetic ulcers, pressure ulcers, or venous insufficiency ulcers, flaps, and grafts, for example. The term "tissue site" may also refer to areas of any tissue that are not necessarily wounded or defective, but are instead areas in which it may be desirable to add or promote the growth of additional tissue.

The term micrograft as used herein may refer to relatively small skin tissue grafts, for example, a skin tissue graft that is smaller than the intended recipient site. For example, the term micrograft may encompass skin tissue grafts that have a width or length less than a millimeter, or less than 100 microns, or from about 1 to about 50 microns. For example, a micrograft may be an excised skin segment having at least one dimension parallel to the skin surface that is less than a millimeter, or less than 100 microns, or less than 10 microns, and the other dimension parallel to the skin surface is less than 500 microns, or less than 100 micrometers, or less than 50 microns, or less than 10 microns, or less than 1 microns. A micrograft may be generally circular in a plane parallel to the skin surface. A micrograft may also have a depth that extends at least through the epidermis and, additionally in some applications, may encompass at least one layer of basal cells. The depth dimension may range from about 500 micrometers to about 0.1 micrometer, or from about 100 micrometers to about 1 micrometer. The terms "generally circular" and "circular" are used interchangeably herein to describe openings that are round, ovalular, oblong, or otherwise form closed polygonal shapes having a major dimension (width or diameter) that is less than 5 times the minor dimension (width or diameter) of the shape. For example, the major dimension may be less than 3 times, or less than 2 times, or less than 1.5 times, the minor dimension. A skin tissue micrograft may be developed and excised or "harvested" using a suitable skin micrograft-harvesting device for example, of the type disclosed herein.

The term harvesting as used herein may refer to and encompass the excision or other removal of one or more skin tissue grafts via the operation of a skin graft generating device, such as, but not limited to, a suction-blister micrograft generator. Harvesting may refer to one or more steps carried out within an overarching process of transplanting one or more skin tissue grafts from a donor site to a recipient site, which may include various intermediate steps, such as culturing, expanding, stretching, treating, or otherwise preparing a skin graft for transfer to the recipient site. Harvesting of skin tissue grafts can be accomplished in many different ways. One common technique for harvesting a skin tissue graft, for example, a skin tissue micrograft, involves the application of suction to separate a surface portion of the skin, for example, the epidermis and a basal cell layer, from the underlying dermis. Harvesting of suction blisters may also involve a heat source to facilitate blister formation.

In some embodiments, the application of the suction forces during harvesting of the blisters may be provided by a negative-pressure source. The term negative pressure as used herein may refer to a pressure less than a local ambient pressure, such as the ambient pressure in a local environment external to a sealed environment. In many cases, the local ambient pressure may also be the atmospheric pressure at which a tissue site is located. Alternatively, the negative pressure may be less than a hydrostatic pressure associated with tissue at the tissue site. A negative-pressure supply may be a reservoir of air at a negative pressure, or may be a manual or electrically-powered device that can reduce the pressure in a sealed volume, such as a vacuum pump, a suction pump, a wall suction port available at many healthcare facilities, or a micro-pump, for example.

### Skin Micrograft-Harvesting Device

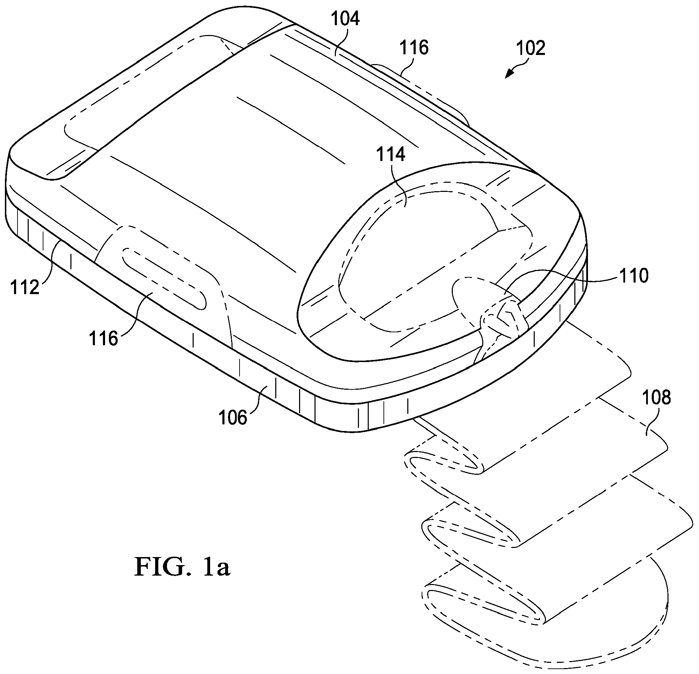

Referring to Figure 1a, an illustrative embodiment of a skin micrograft-harvesting device 102, referred to herein as the "harvester 102", suitable for use in accordance with various aspects of the present disclosure, is shown. In this illustrative embodiment, the harvester 102 may generally include a detachable head portion 104 and a harvester body 106. The harvester body 106 may be adapted for placement on, for example, adjacent to, a patient's skin at a donor site where skin grafts or micrografts are to be obtained. For example, the harvester 102 may be placed on the inner thigh, and may be secured in place, for example, with attachment strap 108 (shown in phantom). In some embodiments, the head portion 104 may further include a heater configured to apply heat to the tissue at the donor site and powered via a coupler 110 adapted to couple with a power source in a base unit. The head portion 104 may further include a seal 112, which may permit a negative pressure chamber to be formed when the head portion 104 and the harvester body 106 are joined together and the harvester 102 is coupled to a vacuum pump or other source of negative pressure, for example, via coupler 110 connecting the harvester 102 to its base unit. The head portion 104 may further include one or more windows 114 for observation of skin blisters, which may be referred to herein as "microdomes," being formed within the chamber by application of negative pressure, heat, or both. The term "microdome" may generally be used herein to refer to a segment of tissue at the donor site, prior to excision. The harvester 102 may be generally configured such that, once the microdomes have been formed, for example, as a result of the application of negative pressure and, optionally, heat, the head portion 104 can be removed. For example, the head portion 104 may be detached by deactivating the source of negative pressure and decoupling the head portion 104 from the harvester body 106, for example, by actuation of release levers 116, which break the seal 112 and allow the head portion 104 to be lifted off of the harvester body 106.

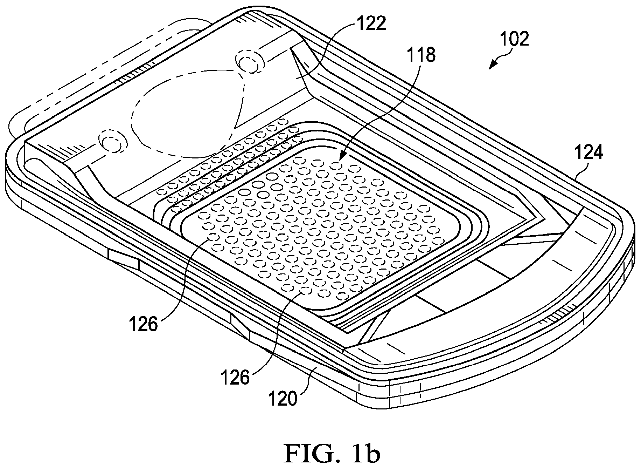

Referring to Figure 1b, a view of the harvester 102 of Fig. 1a is shown with the head portion 104 removed and a cutting mechanism 118 exposed. The harvester body 106 may include a base portion 120, a sled 122, and an actuator handle 124. The cutting mechanism 118 may include a plurality of plates, each having a plurality of holes. The plates of the cutting mechanism may be movable (e.g., horizontally movable) with respect to each other, such that the holes of the various plates may be aligned or misaligned. For example, below the top plate *(e.g.,* a relatively upper-most plate) depicted in Figure 1b, the cutting mechanism 118 may include one or more additional plates, for example, a cutter plate (e.g., an intermediate plate) and a bottom plate *(e.g.,* a relatively lower-most plate), each having holes that may be selectively aligned or misaligned with respect to the holes of the top plate. With the holes 126 initially aligned, and with the head portion 104 joined to the harvester body 106 and activated as disclosed herein, skin at the donor site may be drawn through the holes 126 by heat and/or application of negative pressure, thereby forming the microdomes. Once the microdomes are formed, they may be cleaved via the operation of the cutting mechanism 118. For example, upon actuation of the actuator handle 124, the sled 122 may be caused to move horizontally such that one of the plates below the top plate, for example, the cutter plate may be caused to move horizontally with respect to the top and bottom plates such as via a linkage to the sled 122, thereby occluding the alignment of holes 126 and cleaving the raised blisters or microdomes from the skin donor site, thereby forming the skin micrografts.

Various additional or alternative devices may likewise be suitable for generating and/or harvesting skin micrografts and, as such, the instant disclosure should not be construed as having applicability limited to any particular skin micrograft-harvesting device. An example of a skin micrograft-harvesting device is the Cellutome™ skin harvester, commercially available from Acelity, Inc. of San Antonio, Texas. The Cellutome™ device may be a useful tool for harvesting epithelium, for example, in wound healing applications. The Cellutome™ device provides a way to harvest epidermal skin grafts or micrografts consistently, reliably, and with an automated or semi-automated and relatively painless methodology. These micrografts may be comprised of keratinocytes and melanocytes which may be capably employed in re-epithelialization and repigmentation, respectively. These micrografts may then be transferred onto an intended wound/donor site by the user. Once placed onto the granulated wound bed, the keratinocytes migrate from the micrografts onto the surrounding granulation tissue and begin the process of re-epithelializing the surrounding tissue. Not intending to be bound by theory, this outgrowth may occur in concentric circles from the micrografts and may allow the wound to be fully re-epithelialized more quickly than occurs when epithelialization is solely occurring from the wound margins.

Additional details of harvesters suitable for generating and/or harvesting skin micrografts can be found in U.S. patent application Ser. No. 13/839,518 filed Mar. 15, 2013 (published as US20130204273); U.S. patent application Ser. No. 13/346,329 filed Jan. 9, 2012 (published as US2013145596); U.S. patent application Ser. No. 13/,436,318 also filed Jan. 9, 2012 (published as US2012172894); U.S. patent application Ser. No. 13/014,737 filed Jan. 27, 2011 (published as US2012197267); U.S. patent application Ser. No. 12/851,656 filed Aug. 6, 2010 (published as US2012035599); U.S. patent application Ser. No. 12/851,621 filed Aug. 6, 2010 (published as US2012035618); U.S. patent application Ser. No. 12/851,703 filed Aug. 6, 2010 (published as US2012035620); and U.S. patent application Ser. No. 12/851,682 filed Aug. 6, 2010 (published as US2012035619).

### Substrate

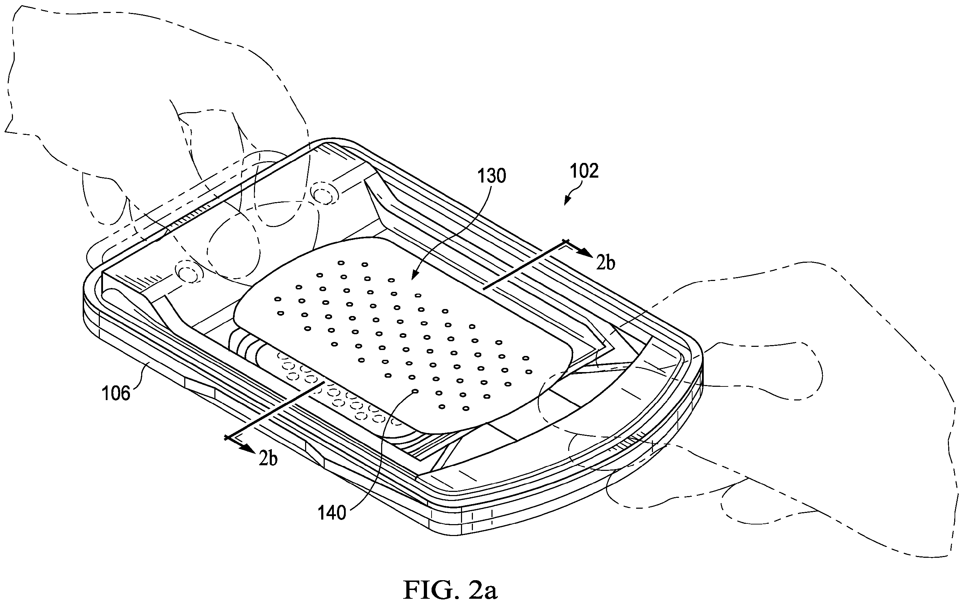

Typically, the substrate, which may also be referred to as a support or transfer substrate, is adapted to collect the micrografts (that is, the excised microdomes), for example, such that the micrografts may be transferred away from the harvester. The substrate may then be applied to a recipient site so that the plurality of micrografts can be assimilated as transplanted tissue. Referring to Figure 2a, the harvester 102 of Figure 1a is shown together with a substrate 130 positioned with respect to the harvester 102 and adapted, for example, for supporting and/or transferring skin micrografts from a donor site to a recipient site.

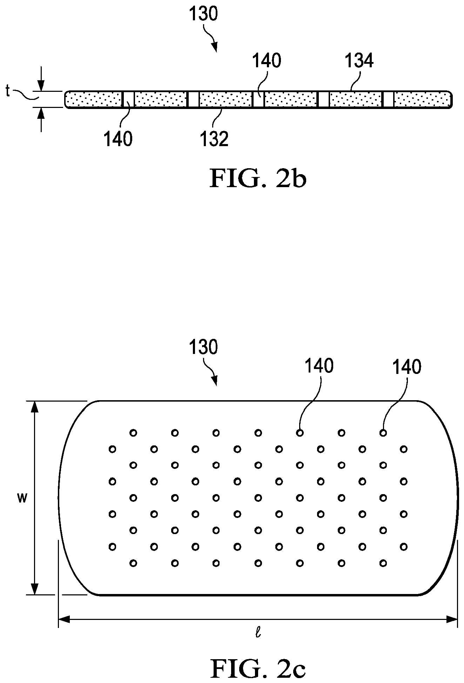

Referring to Figure 2b, a cross-sectional view of the substrate 130 of Fig. 2a is shown and, referring to Figure 2c, a top view of the substrate 130 of Figure 2a is shown. As illustrated, the substrate 130 generally includes a first planar surface 132 and a second substantially planar surface 134. As will be appreciated by one of ordinary skill in the art upon viewing this disclosure, planar surfaces may include those surfaces that, although not necessarily perfectly flat, are generally recognized as flat or capable of being laid flat; for example, a generally planar surface may include minor undulations and/or deviations. Generally, the substrate 130 is sized and shaped suitably as to be received by the harvester 102, for example, such that one of the first substantially planar surface 132 or the second substantially planar surface 134 substantially covers the top plate of the cutting mechanism 118. For example, in the embodiment of Figures 2a, 2b, and 2c, the first and second substantially planar surfaces, 132 and 134, respectively, of the substrate 130 may be characterized as outlining a generally ovalular or stadium shape, although in additional or alternative embodiments the substrate 130 may take any suitable shape or outline. For example, depending upon the harvester with which a given substrate is intended to be employed, a substrate may be circular, oblong, ovalular, rectangular, square, trapezoidal, or any other suitable shape. Likewise, the substrate may have any suitable length, /, and width, w, depending upon the harvester with which a given substrate is intended to be employed. Also, the substrate 130 may have a suitable thickness, *t.* In some embodiments, the length, *l*, and width, w, may be from about 1 centimeters (cm) to about 6 centimeters, for example, 5 cm by 5 cm or 5 cm by 2.5 cm. In some embodiments, the thickness (*t*) of the substrate 130 is within a range of from about 400 microns (µm) to about 2,000 microns (µm). In a more specific embodiment, the thickness of the substrate 130 is within a range of from about 450 microns (µm) and about 1,500 microns (µm) or from about 500 microns (µm) and about 1,000 microns (µm). For example, the substrate 130 may comprise or be characterized as a film and/or a thin film.

### Transfer Substrate - Composition

In some embodiments, the substrate 130 may be formed from a composition comprising one or more of constituents of an intended end product, such as the substrate 130, for example, a composition comprising a mixture of the at least of some of the constituents of the intended end product, referred to herein as the "substrate-forming composition." In some embodiments, the substrate-forming composition, which may be characterized as a slurry, may be characterized as having from about 0.5% to about 3.0% solids content, more particularly, as having about 1.0% solids content. In one or more of the embodiments disclosed herein, the substrate 130 may be characterized with respect to the end product, for example, the substrate 130, itself, the substrate-forming composition, or both.

In some embodiments, the substrate 130 may be characterized as being bioresorbable and/or as exhibiting bioresorbability. As used herein, the terms "bioresorbable" and "bioresorbability" may refer to a characteristic of a material to disintegrate, degrade, or dissolve upon exposure to physiological fluids or processes, for example, as will be disclosed herein, when the substrate is positioned with respect to a tissue site. It is understood that such bioresorbability may be exhibited as a result of chemical process or condition, a physical process or condition, or combinations thereof.

A bioresorbable material and/or a structure formed from a bioresorbable material (e.g., the substrate 130) may be configured to exhibit a particular proportion of disintegration, degradation, or dissolution within a particular time period. For instance, in various embodiments the substrate 130 may be configured such that about 70% by weight, about 80% by weight, about 90% by weight, or about 95% by weight, or about 99% by weight, or about 100% by weight of the substrate 130 may be disintegrated, degraded, or dissolved with in a time period of from about 10 days to about 8 hours, or from about 7 days to about 12 hours, or from about 5 days to about 24 hours, from introduction into a physiological environment and/or when incubated with simulated physiological fluid at a temperature of about 37° C.

In any embodiment herein, the substrate 130 and/or the substrate-forming composition may comprise one or more suitable bioresorbable materials. Examples of suitable bioresorbable materials include, but are not limited to, collagen, oxidized cellulose, oxidized regenerated cellulose (ORC), gelatin, alginates, chitosan, chitin, guar gums, pectin, starch derivatives, cellulose derivatives (such as hydroxyethyl cellulose, hydroxypropyl cellulose, and hydroxypropylmethyl cellulose), glycosaminoglycans, galactomannans, chondroitin salts (such as chondroitin sulfate), heparin salts (such as heparin sulfate), hyaluronic acid and salts thereof, hyaluronates, and combinations of any two or more thereof.

In any embodiment herein, the collagen may be obtained from any suitable natural source. The collagen may be Type I, II or III collagen, or may also be chemically modified collagen, for example an atelocollagen obtained by removing the immunogenic telopeptides from natural collagen. The collagen may also comprise solubilized collagen or soluble collagen fragments (obtained, for example, by pepsin treatment of a natural collagen) having a weight-average molecular weight of about 5,000 to about 100,000; thus, the collagen may have a weight-average molecular weight of about 5,000, about 6,000, about 7,000, about 8,000, about 9,000, about 10,000, about 12,000, about 14,000, about 16,000, about 18,000, about 20,000, about 22,000, about 24,000, about 26,000, about 28,000, about 30,000, about 32,000, about 34,000, about 36,000, about 38,000, about 40,000, about 45,000, about 50,000, about 55,000, about 60,000, about 65,000, about 70,000, about 75,000, about 80,000, about 85,000, about 90,000, about 95,000, about 100,000, or any range including and/or in between any two of these values. As understood by one of ordinary skill in the art, "molecular weight" (also known as "relative molar mass") is a dimensionless quantity but is converted to molar mass by multiplying by 1 gram/mole - for example, collagen with a weight-average molecular weight of 5,000 has a weight-average molar mass of 5,000 g/mol. The collagen may be obtained from bovine corium that has been rendered largely free of non-collagenous components. Such non-collagenous components include fat, non-collagenous proteins, polysaccharides and other carbohydrates, as described in U.S. Patent 4,614,794, Easton et al., issued September 30, 1986 and U.S. Patent 4,320,201, Berg et al., issued March 16, 1982.

In any embodiment herein, the collagen may be present in the substrate 130 in an amount suitable to impart a desired characteristic to the substrate 130. For example, collagen may be present in the substrate 130 in an amount from about 40% to about 90% by weight, or from about 50% to about 60% collagen by weight of the substrate 130. Thus, the collagen may be present by weight of the substrate 130 at about 50%, about 52%, about 54%, about 56%, about 58%, about 60%, or any range including and/or in between any two of these values. Additionally, in some embodiments, the collagen may be present in the substrate-forming composition in an amount from about 40% to about 90% by weight, or from about 50% to about 60% by weight of the solids content of the substrate-forming composition, for example, from about 0.2% to about 2.7% by weight of a substrate-forming composition having a solids content of from 0.5% to about 3.0% by weight.

In any embodiment herein, the oxidized cellulose may be produced via the oxidation of cellulose, for example, with dinitrogen tetroxide. Not intending to be bound by theory, treatment via dinitrogen tetroxide may convert primary alcohol groups on the saccharide residues to carboxylic acid groups, forming uronic acid residues within the cellulose chain. This oxidation may not proceed with complete selectivity, and as a result, hydroxyl groups on carbons 2 and 3 may be converted to the keto- form. These ketone units introduce an alkali labile link, which at a pH of about 7 or higher, may be effective to initiate the decomposition of the polymer via formation of a lactone and sugar ring cleavage. As a result, an oxidized cellulose may be biodegradable and bioabsorbable under physiological conditions.

In any embodiment herein, the oxidized regenerated cellulose ("ORC") may be prepared by oxidation of a regenerated cellulose, such as rayon. ORC may be manufactured via any suitable process, for example, by the process described in U.S. Patent 3,122,479. The ORC may have a suitable degree of oxidation and, hence, a suitable rate of degradation.

In any embodiment herein, the ORC may be in any suitable physical form, including but not limited to particles, fragments, and powders. For example, the ORC may be in the form of water-soluble low molecular weight fragments obtained by the alkaline hydrolysis of ORC. Additionally or alternatively, the ORC may be in the form of particles, such as fiber particles or powder particles. In various embodiments, the ORC may be fibers, and the ORC fibers may be present in a volume fraction such that at least 80% of the fibers have lengths in the range of from about 20 µm to about 50 mm, or such that at least 80% of the fibers have lengths in the range of from about 5 µm to about 1000 µm, or such that at least 80% of the fibers have lengths in the range of from about 250 µm to about 450 µm, or such that at least 80% of the fibers have lengths in the range of from about 25 mm to about 50 mm. Desired size distributions can be achieved, for example, by milling an ORC cloth, followed by sieving the milled powder to remove fibers outside the range.

In any embodiment herein, the oxidized cellulose or ORC may be present in the substrate 130 in an amount suitable to impart a desired characteristic to the substrate 130. For example, the oxidized cellulose or ORC may be present in the substrate 130 in an amount from about 5% to about 45% by weight, or from about 35% to about 45% ORC by weight of the substrate 130. Thus, the percent of oxidized cellulose or ORC by weight of the substrate may be about 35%, about 36%, about 37%, about 38%, about 39%, about 40%, about 41%, about 42%, about 43%, about 44%, about 45%, or any range including and/or in between any two of these values. Additionally, in some embodiments, the oxidized cellulose or ORC may be present in the substrate-forming composition in an amount from about 5% to about 45% by weight, or from about 35% to about 45% oxidized cellulose or ORC by weight of the solids content of the substrate-forming composition, for example, from about 0.025% to about 1.35% by weight of a substrate-forming composition having a solids content of from 0.5% to about 3.0% by weight. In any embodiment herein, a weight ratio of collagen to ORC may be about 60:40, about 59:41, about 58:42, about 57:43, about 56:44, about 55:45, about 54:46, about 53:47, about 52:48, about 51:49, about 50:50, about 49:51, about 48:52, about 47:53, about 46:54, about 45:55, about 44:56, about 43:57, about 42:58, about 41:59, about 40:60, or any range including and/or in between any two of these values.

In any embodiment herein, the substrate 130 may be characterized as exhibiting tackiness or adhesion. Generally, tackiness may be understood as the bond strength of a material after a relatively short contact time between the material and another surface, such as less than about 60 seconds. In any embodiment herein, the bond strength of the substrate 130 may exhibit adhesion sufficient to support a load of from about 10 grams to about 45 grams, determined in accordance with STM711, "Test method for the determination of the adhesion of medical adhesives to a substrate," or a load of from about 15 grams to about 40 grams, or a load from about 20 grams to about 35 grams. For example, the substrate 130 of any embodiment herein may be characterized as exhibiting sufficient tackiness or adhesion such that a plurality of microdomes may be transferred, via the substrate 130, from a donor site to a recipient site.

In any embodiment herein, the substrate 130 and/or the substrate-forming composition may include a suitable adhesion agent (an "adhesive"). Generally, an adhesion agent suitable for inclusion within the substrate-forming composition may impart a desired degree of tackiness to the substrate 130 while not substantially disrupting the bioresorbability properties of the substrate 130, as disclosed herein. Examples of suitable adhesion agents include, but are not limited to sugars, for example, glucose. In any embodiment herein, mixtures such as honey, which may have about 80% sugar content by weight, may be employed. In any embodiment herein, the adhesion agent may be incorporated within the substrate-forming composition in an amount suitable to impart a desired tackiness to the substrate 130. In any embodiment herein, the adhesion agent (*e.g.,* one or more sugars) may be present within the substrate 130 in an amount (by weight of the substrate 130) of about 1%, about 2%, about 3%, about 4%, about 5%, about 6%, about 7%, about 8%, about 9%, about 10%, about 11%, about 12%, about 13%, about 14%, about 15%, about 16%, about 17%, about 18%, about 19%, about 20%, or any range including and/or in between any two of these values. In any embodiment herein, glucose may be present within the substrate 130 in an amount (by weight of the substrate 130) of about 8%, about 9%, about 10%, about 11%, about 12%, about 13%, about 14%, about 15%, about 16%, or any range including and/or in between any two of these values. In any embodiment herein, the glucose may be present within the substrate-forming composition in an amount of from about 4 grams to about 8 grams per 50 grams of a substrate-forming composition having a solids content of about 1% by weight.

In alternative embodiments to those described in the paragraph above, an adhesion agent may be applied to a substrate, for example, a substrate that does not itself exhibit adhesion, as disclosed herein. For example, the substrate may comprise an adhesive layer applied to a substrate that, by itself, does not exhibit significant adhesive properties. In such an embodiment, the adhesive layer may comprise any suitable adhesive composition, for example, comprising glucose. Alternatively, the adhesive layer may comprise any suitable adhesive, for example, a medically-acceptable adhesive suitable to capture skin micrografts and/or adhere the substrate to a patient at the transplantation site. Examples of suitable medically-acceptable adhesives include, but are not limited to, acrylic adhesives, rubber adhesives, high-tack silicone adhesives, and polyurethane adhesives. The adhesive may be a pressure-sensitive adhesive. In such embodiments, the adhesive layer may be applied so as to impart a desired degree of tackiness to the substrate. Factors that may be manipulated to control the tackiness of the adhesive layer include the thickness of the adhesive layer and the amount (e.g., concentration) of the adhesive within the adhesive layer. For example, the adhesive layer may have a thickness of about 30 microns.

The adhesive layer may be a continuous layer or a discontinuous layer. For example, substantially all (e.g., at least about 90%, 95%, 99%, or 100%) of at least one of the first and second substantially planar surfaces may be coated (e.g., treated) with the adhesive composition to form the adhesive layer. Alternatively, the adhesive composition may be applied to at least one of the first and second substantially planar surfaces in a plurality of patches, for example, substantially corresponding to (e.g., aligned with) the position of each of the holes 126 of the cutting mechanism 118 of the harvester 102 when the substrate is positioned within the harvester 102 and, for example, thereby substantially proximate to the microdomes and/or micrografts.

In any embodiment herein, the substrate 130 and/or the substrate-forming composition may comprise one or more additional, optional materials. Such optional components may include, for example, preservatives, stabilizing agents, hydrogels and other gelling agents, plasticizers, matrix strengthening materials, dyestuffs, and actives.

In any embodiment herein, the substrate 130 and/or the substrate-forming composition may comprise a plasticizer. An example of such a plasticizer is glycerol. Glycerol may be included in any suitable amount so as to impart a desired plasticity to the substrate 130. For example, the glycerol may be present within the substrate-forming composition in an amount of from about 0.5 microliters (µl) to about 4 microliters (µl) per 50 grams of substrate-forming composition, or from about 1 microliters (µl) to about 3 microliters (µl) per 50 grams of substrate-forming composition, or about 2 microliters (µl) per 50 grams of the substrate-forming composition.

The substrate 130 and/or the substrate-forming composition may comprise an optional gelling agent, such as a hydrogel. Such gelling agents include those selected from the group consisting of polyurethane gels, modified acrylamide polymers, and hydrophilic polysaccharides. Such hydrophilic polysaccharides useful herein include alginates, chitosan, chitin, guar gums, pectin, starch derivatives, cellulose derivatives (such as hydroxyethyl cellulose, hydroxypropyl cellulose, and hydroxypropylmethyl cellulose), glycosaminoglycans, galactomannans, chondroitin salts (such as chondroitin sulfate), heparin salts (such as heparin sulfate), hyaluronic acid and salts thereof, hyaluronates, and mixtures of any two or more thereof.

In any embodiment herein, the substrate 130 and/or the substrate-forming composition may comprise carboxymethylcellulose ("CMC"), for example, to modify the rheological, absorbency, and other characteristics of the substrate 130 and/or the substrate-forming composition. The CMC may be derived from cellulose and modified such that carboxymethyl groups are bonded to hydroxyl groups in the glucopyranose monomers that make up the cellulose. The CMC may be in salt form, comprising a physiologically acceptable cation, such as sodium (i.e., sodium CMC). CMC is commercially available as Walocel™ (sold by The Dow Chemical Company) and Cekol® (sold by CP Kelco). The CMC may be present in the substrate-forming composition at any level appropriate to result in the desired characteristics.

In any embodiment herein, the substrate 130 and/or the substrate-forming composition may comprise a strengthening material, for example, to improve the handling characteristics of the substrate by, for example, decreasing its susceptibility to tearing. An example of a suitable strengthening material includes non-gelling cellulose fibers. Such "non-gelling" cellulose fibers may be substantially water-insoluble, and may be produced from cellulose that has not been chemically modified to increase water solubility (as contrasted from CMC or other cellulose ethers). Non-gelling cellulose fibers are commercially available as Tencel® fibers (sold by Lenzing AG). Such fibers may be processed from a commercially-available continuous length, by cutting into lengths that are, in some embodiments, from about 0.5 to about 5 cm, or from about 2 to about 3 cm in length. The non-gelling cellulose fibers may be present in the substrate 130 and/or the substrate-forming composition at any level appropriate to result in the desired physical characteristics of the substrate 130.

In any embodiment herein, the substrate 130 and/or the substrate-forming composition may also comprise one or more active ingredients which aid in wound healing. Active ingredients include non-steroidal anti-inflammatory drugs, acetaminophen, steroids, optional antibiotics and antiseptics (e.g., silver and chlorhexidine), and growth factors (e.g. fibroblast growth factor or platelet derived growth factor). If present, active ingredients are present in "safe and effective" amounts. Such safe and effective amounts are sufficient to impart a desired effect (e.g., antimicrobial activity), without undue adverse side effects (such as toxicity, irritation, or allergic response), commensurate with a reasonable benefit/risk ratio when used in the manner of this technology. The specific safe and effective amount of an active may vary with the active and other factors such as the physical form of the active, the type and quantity of other materials in the composition, the intended use, and the physical condition of the patient on whom the substrate is intended for use. In general, such actives are optionally present at a level of from about 0.1% to about 10%.

As an example, the substrate 130 and/or the substrate-forming composition may comprise a growth factor. Examples of suitable growth factors include, but are not limited to, platelet derived growth factor (PDGF), fibroblast growth factor (FGF), and epidermal growth factor (EGF), and mixtures thereof.

As another example, the substrate 130 and/or the substrate-forming composition may comprise an antimicrobial agent, an antiseptic, or both. Examples of antimicrobial agents include, but are not limited to, tetracycline, penicillins, terramycins, erythromycin, bacitracin, neomycin, polymycin B, mupirocin, clindamycin, and combinations thereof. Examples of antiseptics include, but are not limited to silver, polyhexanide (polyhexamethylene biguanide or PHMB), chlorhexidine, povidone iodine, triclosan, sucralfate, quaternary ammonium salts, and combinations of any two or more thereof. For example, in any embodiment herein, the substrate 130 may comprise silver, which may be in metallic form, in ionic form (e.g., a silver salt), or both. For example, the silver may be present in ionic form. In any embodiment herein, the substrate 130 may comprise a complex of silver and ORC (a "Silver/ORC complex"). As referred to herein, such a complex is an intimate mixture at the molecular level, for example, with ionic or covalent bonding between the silver and the ORC. The Silver/ORC complex may comprise a salt formed between the ORC and Ag⁺, but it may also comprise silver clusters or colloidal silver metal, for example produced by exposure of the complex to light. The complex of an anionic polysaccharide and silver contained in the materials of the present invention can be made by treating the ORC with a solution of a silver salt. In any embodiment herein, the silver salt may be the salt of silver with a weak acid. Silver/ORC complexes useful herein, and methods of producing such complexes, are described in U.S. Patent 8,461,410, Cullen et al., issued June 11, 2013. Similar processes are described in U.S. Patent 5,134,229, Saferstein et al., issued July 28, 1992. In any embodiment herein, the Silver/ORC Complex may be present in the substrate 130 at a level of from about 1% to about 2% by weight, and the Silver/ORC Complex may comprise from about 20% to about 30% (e.g., about 25%) of silver by weight of the ORC. Alternatively, in any embodiment herein, it may be the substrate 130 and/or the substrate-forming composition does not contain an antimicrobial agent or an antiseptic.

In any embodiment herein, such as where the substrate 130 comprises silver, the substrate 130 and/or the substrate-forming composition may comprise a dyestuff. The dyestuff may be light-absorbing in the visible region 400-700 nm. Such dyestuffs may be operable to photochemically trap generated free radicals that could otherwise react with the silver in the present compositions, acting as photochemical desensitisers. In any embodiment herein, the antioxidant dyestuff may be selected from the group consisting of aniline dyes, acridine dyes, thionine dyes, bis-naphthalene dyes, thiazine dyes, azo dyes, anthraquinone dyes, and mixtures of any two or more thereof. For example, the antioxidant dyestuff may be selected from the group consisting of gentian violet, aniline blue, methylene blue, crystal-violet, acriflavine, 9-aminoacridine, acridine yellow, acridine orange, proflavin, quinacrine, brilliant green, trypan blue, trypan red, malachite green, azacrine, methyl violet, methyl orange, methyl yellow, ethyl violet, acid orange, acid yellow, acid blue, acid red, thioflavin, alphazurine, indigo blue, methylene green, and mixtures thereof. If present, the dyestuff may be present in the substrate 130 at a level of about 0.05% to about 5%, typically about 0.2% to about 2% by weight of the substrate 130.

In any embodiment herein, the substrate 130 may be essentially free of water. In any embodiment herein, the substrate-forming composition may comprise up to 20% water. In any embodiment herein, the substrate 130 may contain 10% or less, 8% or less, or 5% or less, of water. In any embodiment herein, the substrate 130 may be freeze-dried, for example, such as through lyophilization.

By way of example, in any embodiment herein the substrate 130 may be characterized as exhibiting bioresorbability and as exhibiting tackiness. The substrate 130 may be formed from a substrate-forming composition comprising collagen, ORC, glucose, and glycerol. In such an embodiment, the substrate-forming composition may comprise from about 0.5% to about 3.0% solids, more particularly, about 1% solids. Collagen may be present within the substrate-forming composition in an amount from 0.2% to about 2.7% by weight of the substrate-forming composition. ORC may be present within the substrate-forming composition in an amount from 0.025% to about 1.35% by weight of the substrate-forming composition. Glucose may be present in the substrate-forming composition in an amount from about 4 grams to about 8 grams per 50 grams of substrate-forming composition, or from about 5 grams to about 7 grams per 50 grams of substrate-forming composition. Glycerol may be present within the substrate-forming composition in an amount from about 0.5 microliters (µl) to about 4 microliters (µl) per 50 grams of substrate-forming composition, or from about 1 microliter(µl) to about 3 microliters (µl) per 50 grams of substrate-forming composition, or about 2 microliters (µl) per 50 grams of substrate-forming composition. The substrate-forming composition may be formed into a suitable size and shape (e.g., poured, molded, trimmed), for example, to the form the substrate 130. Additionally, the substrate may be freeze-dried, for example, through lyophilization. The substrate 130 may comprise collagen in an amount from about 50% to about 60% by weight, ORC in an amount from about 35% to about 45% by weight, glucose in an amount from about 8% to about 16% by weight, and glycerol in an amount resulting from the incorporation of glycerol within the substrate-forming composition at the disclosed rates.

In more particular embodiments, the substrate 130 may be characterized as exhibiting bioresorbability and as exhibiting tackiness. The substrate 130 may be formed from a substrate-forming composition comprising collagen, ORC, glucose, glycerol, and silver. In such an embodiment, the substrate-forming composition may comprise from about 0.5% to about 2.0% solids, more particularly, about 1% solids. Collagen may be present within the substrate-forming composition in an amount from about 0.2% to about 2.7% by weight of the substrate-forming composition. ORC may be present within the substrate-forming composition in an amount from 0.025% to about 1.35% by weight of the composition. Glucose may be present in the substrate-forming composition in an amount from about 4 grams to about 8 grams per 50 grams of substrate-forming composition, or from about 5 grams to about 7 grams per 50 grams of substrate-forming composition. Glycerol may be present within the substrate-forming composition in an amount from about 0.5 microliters (µl) to about 4 microliters (µl) per 50 grams of substrate-forming composition, or from about 1 microliter (µl) to about 3 microliters (µl) per 50 grams of substrate-forming composition, or about 2 microliters (µl) per 50 grams of substrate-forming composition. Silver may be present within the substrate-forming composition in an amount from about 0.005% to about 0.06% by weight of the composition. The substrate-forming composition may be formed into a suitable size and shape (e.g., poured, molded, trimmed), for example, to the form the substrate 130. Additionally, the substrate may be freeze-dried, for example, through lyophilization. The substrate 130 may comprise collagen in an amount from about 50% to about 60% by weight, ORC in an amount from about 35% to about 45% by weight, glucose in an amount from about 8% to about 16% by weight, glycerol in an amount resulting from the incorporation of glycerol within the substrate-forming composition at the disclosed rates, and silver present as a Silver/ORC complex in an amount from about 1% to about 2% by weight of the substrate 130.

In any embodiment herein, the substrate 130 may be fluid permeable. For instance, referring to Figures 2a, 2b, and Fig. 2c, the substrate 130 may be porous, for example, comprising a plurality of pores 140 disposed within the substrate 130 and extending between the first substantially planar surface 132 and the second substantially planar surface 134 of the substrate 130. The term "porous" as used herein may encompass not only defined apertures or holes (such as pores 140) but also permeable and open cell foam structures, a network of lines, incisions, or other openings adapted to provide fluid communication between the first substantially planar surface 132 and the second substantially planar surface 134 of the substrate 130.

The pores 140 may have any suitable cross-sectional shape, for example, being generally square, rectangular, circular, ovalular, or elongated. Generally, the pores 140 are adapted to allow fluid communication between the first and second substantially planar surfaces of the substrate, for example, so as to allow fluid such as exudate to be communicated from a tissue site through the substrate 130. The diameter of the pores 140 may vary. For example, the average diameter of the pores 140 may be from about 2 mm to about 10 mm, or from about 4 mm to about 8 mm.

The pores 140 may be present within the substrate 130 in a uniform pattern or may be randomly distributed on the substrate 130. For example, the pores 140 may be positioned within the substrate 130 such that, when the substrate 130 is positioned within the harvester 102, the pores do not correspond to (e.g., are not aligned with) the position of each of the holes 126 of the cutting mechanism 118 of the harvester 102. For example, the pores 140 may be positioned within the substrate 130 such that, when the substrate 130 is positioned within the harvester 102, the pores 140 are substantially spaced between the holes 126, for example, such that a given pore is substantially equidistant with respect to two or more adjacent holes 126 of the harvester 102.

In any embodiment herein, the substrate 130 may be characterized as transparent or substantially transparent. For example, the transparency or substantial transparency of the substrate 130 may allow objects, such as microdomes or micrografts, to be perceived through the substrate 130. The term "substantially transparent" as used herein will be understood by persons of ordinary skill in the art and will vary to some extent depending upon the context in which it is used. If there are uses of the term which are not clear to persons of ordinary skill in the art, given the context in which it is used, the term means that the material allows a light transmission of about 74% or more of a beam of light having a wavelength of 400 nm directed to the material at a specular angle of 10° through a thickness of 2 mm of the material.

### Methods

Also disclosed herein are one or more methods for transplanting a tissue micrograft, for example, using a composition, for example, in the form of a substrate like substrate 130 and/or a substrate-forming composition. Generally, a method for transplanting a tissue micrograft may comprise the steps of forming a tissue microdome, excising the tissue microdome from the donor site to form the tissue micrograft, and transplanting the tissue micrograft from the donor site to a recipient site with a substrate like the substrate 130 disclosed herein, for example, a substrate having a first surface and a second surface, and comprising a bioresorbable material and an adhesive.

In any embodiment herein, to form the tissue microdome, such as a skin microdome, a user such as a physician, clinician, or other caregiver, may begin by preparing donor site. As will be appreciated by those of ordinary skill in the art upon viewing this disclosure, any suitable donor site may be selected, for example, depending upon various factors. Examples of suitable tissue donor sites may include but are not limited to, the inner thigh, abdomen, and buttocks. The user may treat or clean the tissue at the donor site, such as by applying an antiseptic. The user may then position the harvester 102 and, in some embodiments, secure the harvester 102 in place, for example, via the attachment strap 108. With the harvester 102 in place and ready for use, the user may operate harvester 102 to form the microdome or a plurality of microdomes which, ultimately, will yield the micrografts. In any embodiment herein, operating the harvester 102 so as to form the microdomes may entail the application of negative pressure, heat, or both to the tissue at the donor site, at sufficient intensities and duration to form the microdomes.

In any embodiment herein, with the microdomes formed, the user may place the substrate 130 in the harvester 102 such that one of the first or second substantially planar surfaces faces upwards (e.g., away from the harvester body 106) and the other faces downward (e.g., toward the harvester body 106), more particularly, so as to be in contact with the top plate of the cutting mechanism 118. By so-placing the substrate 130, one of the first or second substantially planar surfaces may also come into contact with the skin microdomes formed as disclosed herein. In any embodiment herein, the substrate 130 is so situated, for example, positioned within the harvester body 106, before the cutting mechanism 118 is actuated to cleave the blisters or microdomes into skin micrografts. In alternative embodiments, the substrate 130 may be positioned within the harvester body 106 after cleavage or excision of the microdomes to form the skin micrografts, for example, so as to capture micrografts that have already been excised from the skin. In either event, the substrate 130 will have the micrografts adhered thereto such that the micrografts can then be removed from the harvester body 106 via the substrate 130 and applied to a recipient site.

Ultimately, the substrate 130 containing the plurality of harvested micrografts may be applied to a recipient site of a patient. In any embodiment herein, the size of the area at the recipient site may be about the same size as the area of the substrate 130 having micrografts adhered thereto, or the size of the recipient site may be greater than the area of the substrate 130, in which instance the user may decide to utilize two or more substrates having harvested micrografts from one or more donor sites. Prior to applying the harvested micrografts to the recipient site, the site may be prepared to receive the grafts using a technique known in the art to be suitable. For example, necrotic, fibrotic, or avascular tissue may be removed. The technique used to prepare the site will depend upon various factors, for example, upon the extent of damage to the recipient site. For example, damaged or undesired epidermal tissue, if present at the recipient site, can be removed to prepare the area for receiving the micrografts. In some instances, burned or ulcerated sites may not necessitate removal of epidermal tissue, although some cleaning of the site or other preparation of the site may be performed. Wounds may be debrided and then allowed to granulate for a period of time, for example, several days, prior to application of the graft. Generally, most of the granulation tissue may be removed, for example, since it has a tendency to harbor bacteria.

In any embodiment herein, the substrate 130 having the plurality of micrografts may be placed over the recipient site, for example, such that the substrate 130 may form a dressing. More particularly, the portion of the substrate 130 having the micrografts can be positioned over the area to be repaired or a portion thereof, for example, an area from which the epidermal tissue has been debrided or removed such as for healing or repigmentation. The substrate 130 can be fixed in place over the treatment area, for example, employing the adhesive tendencies of the substrate 130 or, additionally, tape or the like. Once applied to the recipient site, for example, via the substrate 130, the micrografts may expand and coalesce to complete the healing process.

### Benefits

Without limiting the scope or function of the present technology, the systems, compositions (for example, in the form of a substrate), and methods described herein may provide significant benefits, and may provide advantages relative to some systems known in the art, for example, when employed in the context of a tissue transplant procedure such as a skin micrograft transplant procedure. The substrate 130 provides an effective means to transfer harvest micrografts, more particularly, skin mirografts from a donor site to a recipient site. For example, the adhesive characteristics of the substrate may be effective to adhere the harvested micrografts during a transplant procedure, as disclosed herein.

Also for instance, and as disclosed herein, the substrate 130 is adapted to be resorbed after sufficient time has elapsed to allow attachment and growth of the micrografts at the recipient site, for example, in the range of from several days to a few weeks. In some embodiments, the bioresorbable characteristics of the substrate 130 allow the substrate 130 to be left in place at the recipient site for substantial periods of time. For example, and not intending to be bound by theory, because the substrate 130 may be bioresorbable, the substrate 130 does not necessitate removal, for example, to avoid in-growth of tissue, so as to ensure that the growing tissue at the recipient site does not become attached to the substrate 130. The capability to leave the substrate 130 in place for longer periods of time yields several advantages. For instance, by leaving the substrate 130 in place, premature removal, potentially resulting in disturbance of or trauma to the transplanted micrografts, may be avoided. Also, by leaving the substrate 130 in place, trauma to recipient tissue site that would otherwise result from removal can be avoided. Additionally, leaving the substrate 130 in place for sustained time periods may yield improved micrograft efficacy, for example, by allowing time for attachment, growth, and flourishment of the micrografts.

Further, the substrate 130 may be effective to modulate protease activity at the recipient site, which may also be beneficial to the transplanted micrografts. For example, and not intending to be bound by theory, the substrate 130 may modulate excessive protease activity, such as matrix metallo protease (MMP) activity, by providing a biologically active, sacrificial proteolytic enzyme substrate. For example, MMPs, such as MMP-2 and MMP-9 may preferentially target the sacrificial proteolytic enzyme substrate, for example, the collagen, which may be effective to reduce the protease activity experienced by the transplanted micrografts.

Further still, the substrate 130 may be effective to allow the movement of fluid therethrough, for example, via the pores 140, which may also be beneficial to the transplanted micrografts.

### EXAMPLES

The present technology is further illustrated by the following Examples, which should not be construed as limiting in any way.

The tissue micrograft-harvesting device disclosed herein will be placed on a donor site in patients that are in need of a tissue graft. The tissue microdomes generated using the micrograft-harvesting device form the tissue micrograft that is then adhered to the substrate of the present technology. The micrografts will then be removed from the harvesting device via the substrate and will be applied to a recipient site in the patients. A control group of patients that do not receive the tissue micrograft will also be included.

It is anticipated that patients that receive the substrate-tissue micrograft composition will exhibit improved healing at the recipient site and reduced trauma at the donor site compared to that observed in patients in the control group.

These results demonstrate that the systems and devices of the present technology are useful in methods for cultivating and transplanting a tissue graft in a subject in need thereof.

### Configuration of Exemplary Embodiments

While shown in a few illustrative embodiments, a person having ordinary skill in the art will recognize that the systems, apparatuses, and methods described herein are susceptible to various changes and modifications. Moreover, descriptions of various alternatives using terms such as "or" do not require mutual exclusivity unless clearly required by the context, and the indefinite articles "a" or "an" do not limit the subject to a single instance unless clearly required by the context. Components disclosed herein as part of a system may be also be combined or eliminated in various configurations for purposes of sale, manufacture, assembly, or use. In some configurations the harvester 102 and/or the substrate 130, as disclosed herein may be separated from each other or other components, such as for manufacture or sale, for example, such that the harvester 102 and the substrate 130 may be manufactured, configured, assembled, or sold independently of each other or various other components.

As used herein, the word "include," and its variants, is intended to be non-limiting, such that recitation of items in a list is not to the exclusion of other like items that may also be useful in the materials, compositions, devices, and methods of this technology. Similarly, the terms "can" and "may" and their variants are intended to be non-limiting, such that recitation that an embodiment can or may comprise certain elements or features does not exclude other embodiments of the present technology that do not contain those elements or features. Moreover, descriptions of various alternatives using terms such as "or" do not require mutual exclusivity unless clearly required by the context, and the indefinite articles "a" or "an" do not limit the subject to a single instance unless clearly required by the context.

Although the open-ended term "comprising," as a synonym of non-restrictive terms such as including, containing, or having, is used herein to describe and claim embodiments of the present technology, embodiments may alternatively be described using more limiting terms such as "consisting of' or "consisting essentially of." Thus, for any given embodiment reciting materials, components or process steps, the present technology also specifically includes embodiments consisting of, or consisting essentially of, such materials, components or processes excluding additional materials, components or processes (for consisting of) and excluding additional materials, components or processes affecting the significant properties of the embodiment (for consisting essentially of), even though such additional materials, components or processes are not explicitly recited in this application. For example, recitation of a composition or process reciting elements A, B and C specifically envisions embodiments consisting of, and consisting essentially of, A, B and C, excluding an element D that may be recited in the art, even though element D is not explicitly described as being excluded herein.

Disclosure of values and ranges of values for specific parameters (such as temperatures, molecular weights, weight percentages, etc.) are not exclusive of other values and ranges of values useful herein. It is envisioned that two or more specific exemplified values for a given parameter may define endpoints for a range of values that may be claimed for the parameter. For example, if Parameter X is exemplified herein to have value A and also exemplified to have value Z, it is envisioned that parameter X may have a range of values from about A to about Z. Similarly, it is envisioned that disclosure of two or more ranges of values for a parameter (whether such ranges are nested, overlapping or distinct) subsume all possible combination of ranges for the value that might be claimed using endpoints of the disclosed ranges. For example, if parameter X is exemplified herein to have values in the range of 1 - 10, or 2 - 9, or 3 - 8, it is also envisioned that Parameter X may have other ranges of values including 1 - 9, 1 - 8, 1 - 3, 1 - 2, 2 - 10, 2 - 8, 2 - 3, 3 - 10, and 3 - 9.

The term "about," as used herein, is intended to refer to deviations in a numerical quantity that may result from various circumstances, for example, through measuring or handling procedures in the real world; through inadvertent error in such procedures; through differences in the manufacture, source, or purity of compositions or reagents; from computational or rounding procedures; and other deviations as will be apparent by those of skill in the art from the context of this disclosure. For example, the term "about" may refer to deviations that are greater or lesser than a stated value or range by 1/10 of the stated value(s), e.g., ±10%, as appropriate from the context of the disclosure. For instance, a concentration value of "about 30%" may refer to a concentration between 27% and 33%. Whether or not modified by the term "about," quantitative values recited in the claims include equivalents to the recited values, for example, deviations from the numerical quantity, as would be recognized as equivalent by a person skilled in the art in view of this disclosure.

As used herein, the terms "individual", "patient", or "subject" can be an individual organism, a vertebrate, a mammal, or a human. In some embodiments, the individual, patient or subject is a human.

## Claims

1. A substrate (130) for transplanting a tissue micrograft, the substrate comprising:
a first surface (132) and a second surface (134);
a bioresorbable material; and
an adhesive;
wherein the substrate is configured to receive the tissue micrograft and be positioned at a recipient site;
**characterized in that** the adhesive comprises a sugar.

2. The substrate of claim 1 further comprising a plasticizer.

3. The substrate of one of claims 1-2, wherein the bioresorbable material comprises glycerol, optionally wherein the bioresorbable material is formed from a composition comprising from about 0.5 microliters of glycerol to about 4 microliters of glycerol per 50 grams of the composition.

4. The substrate of one of claims 1-3, wherein the tissue micrograft comprises skin tissue.

5. The substrate of one of claims 1-4, wherein the tissue micrograft is comprised of microdomes.

6. The substrate of one of claims 1-5, wherein the bioresorbable material comprises oxidized regenerated cellulose (ORC) optionally wherein the bioresorbable material comprises from about 40% to about 50% of the ORC by weight.

7. The substrate of one of claims 1-6, wherein the bioresorbable material comprises collagen, optionally wherein the bioresorbable material comprises from about 50% to about 60% of the collagen by weight.

8. The substrate of one of claims 1-7, wherein the bioresorbable material comprises collagen and ORC.

9. The substrate of one of claims 1-8, wherein the substrate comprises about 1% to about 20% adhesive by weight of the substrate.

10. The substrate of one of claims 1-9, wherein the adhesive comprises glucose, optionally wherein the substrate comprises from about 8% to about 16% glucose by weight.

11. The substrate of one of claims 1-10, wherein the substrate further comprises an antimicrobial agent, optionally wherein the antimicrobial agent comprises ionically-bound silver.

12. The substrate of one of claims 1-11, wherein the substrate comprises a plurality of pores (140) extending between the first surface and the second surface of the substrate, optionally wherein the pores have an average cross-sectional dimension from about 0.1 millimeters to about 2 millimeters.

13. The substrate of one of claims 1-12, wherein the substrate exhibits protease-modulating activity, and optionally wherein the protease is MMP-2 and/or MMP-9.

14. The substrate of one of claims 1-13, wherein the substrate is transparent.

15. A system for transplanting a tissue micrograft, the system comprising:
a tissue micrograft-harvesting device (102) comprising a base-plate having a plurality of apertures (126); and
a substrate (130) of any preceding claim.

16. The system of claim 15, wherein the substrate covers the plurality of apertures of the base plate when the substrate is positioned with respect to the tissue micrograft-harvesting device.

17. The system of any of claims 15-16, wherein the substrate comprises a plurality of pores extending between the first surface and the second surface of the substrate; optionally wherein the pores have an average cross-sectional dimension from about 0.1 millimeters to about 2 millimeters; and wherein the pores of the substrate do not align with the apertures of the base plate when the substrate is positioned with respect to the tissue micrograft-harvesting device.

## Patentansprüche

1. Substrat (130) zum Transplantieren eines Gewebemikrografts, wobei das Substrat umfasst:
eine erste Oberfläche (132) und eine zweite Oberfläche (134);
ein bioresorbierbares Material; und
einen Kleber;
wobei das Substrat dazu ausgebildet ist, das Gewebemikrograft aufzunehmen und bei einer Empfangsstelle positioniert zu werden;
**dadurch gekennzeichnet, dass** der Kleber einen Zucker umfasst.

2. Substrat nach Anspruch 1, weiter umfassend einen Weichmacher.

3. Substrat nach einem der Ansprüche 1-2, wobei das bioresorbierbare Material Glycerol umfasst, optional wobei das bioresorbierbare Material aus einer Zusammensetzung gebildet ist, die etwa 0,5 Mikroliter Glycerol bis etwa 4 Mikroliter Glycerol pro 50 Gramm der Zusammensetzung umfasst.

4. Substrat nach einem der Ansprüche 1-3, wobei das Gewebemikrograft Hautgewebe umfasst.

5. Substrat nach einem der Ansprüche 1-4, wobei das Gewebemikrograft aus Mikrodomes besteht.

6. Substrat nach einem der Ansprüche 1-5, wobei das bioresorbierbare Material oxidierte regenerierte Cellulose (ORC) umfasst, optional wobei das bioresorbierbare Material etwa 40% bis etwa 50% ORC, bezogen auf das Gewicht, umfasst.

7. Substrat nach einem der Ansprüche 1-6, wobei das bioresorbierbare Material Kollagen umfasst, optional wobei das bioresorbierbare Material etwa 50% bis etwa 60% Kollagen, bezogen auf das Gewicht, umfasst.

8. Substrat nach einem der Ansprüche 1-7, wobei das bioresorbierbare Material Kollagen und ORC umfasst.

9. Substrat nach einem der Ansprüche 1-8, wobei das Substrat zu etwa 1% bis etwa 20% Kleber, bezogen auf das Gewicht des Substrats, umfasst.

10. Substrat nach einem der Ansprüche 1-9, wobei der Kleber Glukose umfasst, optional wobei das Substrat etwa 8% bis etwa 16% Glukose, bezogen auf das Gewicht, umfasst.

11. Substrat nach einem der Ansprüche 1-10, wobei das Substrat weiter ein antimikrobielles Mittel umfasst, optional wobei das antimikrobielle Mittel ionisch gebundenes Silber umfasst.

12. Substrat nach einem der Ansprüche 1-11, wobei das Substrat eine Vielzahl von Poren (140) umfasst, die sich zwischen der ersten Oberfläche und der zweiten Oberfläche des Substrats erstrecken, optional wobei die Poren ein Durchschnittsquerschnittsausmaß von etwa 0,1 Millimeter bis etwa 2 Millimeter aufweisen.

13. Substrat nach einem der Ansprüche 1-12, wobei das Substrat Proteasemodulierende Aktivität vorweist, und optional wobei die Protease MMP-2 und/oder MMP-9 ist.

14. Substrat nach einem der Ansprüche 1-13, wobei das Substrat durchsichtig ist.

15. System zum Transplantieren eines Gewebemikrografts, wobei das System umfasst:
eine Gewebemikrograft-Gewinnungsvorrichtung (102), die eine Basisplatte umfasst, die eine Vielzahl von Öffnungen (126) aufweist; und
ein Substrat (130) nach einem vorstehenden Anspruch.

16. System nach Anspruch 15, wobei das Substrat die Vielzahl von Öffnungen der Basisplatte abdeckt, wenn das Substrat in Bezug auf die Gewebemikrograft-Gewinnungsvorrichtung positioniert wird.

17. System nach einem der Ansprüche 15-16, wobei das Substrat eine Vielzahl von Poren umfasst, die sich zwischen der ersten Oberfläche und der zweiten Oberfläche des Substrats erstrecken; optional wobei die Poren ein Durchschnittsquerschnittausmaß von etwa 0,1 Millimeter bis etwa 2 Millimeter aufweisen; und wobei die Poren des Substrats nicht mit den Öffnungen der Basisplatte ausgerichtet sind, wenn das Substrat in Bezug auf die Gewebemikrograft-Gewinnungsvorrichtung positioniert ist.

## Revendications

1. Substrat (130) destiné à la transplantation d'une microgreffe de tissu, le substrat comprenant :
une première surface (132) et une seconde surface (134) ;
un matériau biorésorbable ; et
un adhésif;
dans lequel le substrat est configuré pour recevoir la microgreffe de tissu et être positionné sur un site receveur ;
**caractérisé en ce que** l'adhésif comprend un sucre.

2. Substrat selon la revendication 1 comprenant en outre un plastifiant.

3. Substrat selon l'une des revendications 1-2, dans lequel le matériau biorésorbable comprend du glycérol, facultativement dans lequel le matériau biorésorbable est formé à partir d'une composition comprenant d'environ 0,5 microlitre de glycérol à environ 4 microlitres de glycérol pour 50 grammes de la composition.

4. Substrat selon l'une des revendications 1-3, dans lequel la microgreffe de tissu comprend du tissu cutané.

5. Substrat selon l'une des revendications 1-4, dans lequel la microgreffe de tissu se compose de microdomes.

6. Substrat selon l'une des revendications 1-5, dans lequel le matériau biorésorbable comprend de la cellulose régénérée oxydée (ORC) facultativement dans lequel le matériau biorésorbable comprend d'environ 40 % à environ 50 % d'ORC en poids.

7. Substrat selon l'une des revendications 1-6, dans lequel le matériau biorésorbable comprend du collagène, facultativement dans lequel le matériau biorésorbable comprend d'environ 50 % à environ 60 % de collagène en poids.

8. Substrat selon l'une des revendications 1-7, dans lequel le matériau biorésorbable comprend du collagène et de l'ORC.

9. Substrat selon l'une des revendications 1-8, dans lequel le substrat comprend d'environ 1 % à environ 20 % d'adhésif en poids du substrat.

10. Substrat selon l'une des revendications 1-9, dans lequel l'adhésif comprend du glucose, facultativement dans lequel le substrat comprend d'environ 8 % à environ 16 % de glucose en poids.

11. Substrat selon l'une des revendications 1-10, dans lequel le substrat comprend en outre un agent antimicrobien, facultativement dans lequel l'agent antimicrobien comprend de l'argent liée de manière ionique.

12. Substrat selon l'une des revendications 1-11, dans lequel le substrat comprend une pluralité de pores (140) s'étendant entre la première surface et la seconde surface du substrat, facultativement dans lequel les pores présentent une dimension de section transversale moyenne allant d'environ 0,1 millimètre à environ 2 millimètres.

13. Substrat selon l'une des revendications 1-12, dans lequel le substrat présente une activité de modulation des protéases, et facultativement dans lequel la protéase est la MMP-2 et/ou la MMP-9.

14. Substrat selon l'une des revendications 1-13, dans lequel le substrat est transparent.

15. Système de transplantation d'une microgreffe de tissu, le système comprenant :
un dispositif de récolte de microgreffe de tissu (102) comprenant une plaque de base présentant une pluralité d'ouvertures (126) ; et
un substrat (130) selon une quelconque revendication précédente.

16. Système selon la revendication 15, dans lequel le substrat recouvre la pluralité d'ouvertures de la plaque de base lorsque le substrat est positionné par rapport au dispositif de récolte de microgreffe de tissu.

17. Système selon l'une quelconque des revendications 15-16, dans lequel le substrat comprend une pluralité de pores s'étendant entre la première surface et la seconde surface du substrat ; facultativement dans lequel les pores présentent une dimension de section transversale moyenne allant d'environ 0,1 millimètre à environ 2 millimètres ; et dans lequel les pores du substrat ne s'alignent pas avec les ouvertures de la plaque de base lorsque le substrat est positionné par rapport au dispositif de récolte de microgreffe de tissu.
